# DEMANDE DE BREVET EUROPEEN

(11) **EP 1 174 095 A1**
(43) Date de publication de la demande: **23.01.2002**
(21) Numéro de dépôt: 00810651.0
(22) Date de dépôt: 21.07.2000
(51) Int. Cl.: A61C 13/00, A61K 6/04

(54) **Procédé de réalisation d'une reconstruction dentaire et pièce de reconstruction ainsi obtenue**

(71) Demandeur: METAUX PRECIEUX SA METALOR, CH-2009 Neuchâtel (CH)
(72) Inventeur: Mürrle, Ulrich, 2072 St. Blaise (CH); Steit, Pascal, 1302 Vufflens-la-Ville (CH); Mostardi, André, 3280 Murten (CH); Fischer, Alexandre, 2000 Neuchâtel (CH)
(74) Mandataire: Gresset, Jean

(57) **Abrégé**

L'invention concerne un procédé de réalisation d'une reconstruction dentaire (26), caractérisé en ce qu'il comporte les étapes successives de:
- obtention d'une réplique (16) du moignon de dent à reconstruire en revêtement réfractaire,
- application sur cette réplique d'une pièce de remodelage (18) de la dent comprenant un mélange d'une poudre réfractaire et d'un liquide de dispersion,
- infiltration de ladite pièce au moyen d'un métal en fusion (20), et
- élimination du revêtement réfractaire (16).

## Description

La présente invention se rapporte à l'art dentaire. Elle concerne, plus particulièrement, un procédé de réalisation d'une pièce de reconstruction dentaire ainsi que la reconstruction ainsi obtenue.

Actuellement, la technique la plus couramment utilisée pour fabriquer une reconstruction dentaire est celle de la coulée par cire perdue. Plus précisément, à partir de l'empreinte du dentiste, le technicien réalise une réplique en plâtre ou en résine du moignon à reconstruire puis remodèle la dent à l'aide de cire appliquée sur la réplique. La pièce en cire est ensuite plongée dans un revêtement réfractaire à l'état liquide dont la cuisson permet l'élimination de la cire. Un alliage de reconstruction, non précieux, semi-précieux ou précieux, est alors coulé dans le moule ainsi formé, après quoi il ne reste plus qu'à éliminer le revêtement et les tiges de coulée pour obtenir la pièce de reconstruction et à la travailler en vue de sa mise en bouche (polissage, pose d'un composite de décoration ou d'une céramique).

La présente invention a pur but de fournir un procédé amélioré de fabrication d'une reconstruction dentaire, permettant de réaliser, quasiment à la carte, une pièce dont la composition et les teneurs respectives en éléments non précieux, semi-précieux et précieux peuvent être choisies dans une très large palette.

Pour atteindre ce but, le procédé selon l'invention est caractérisé en ce qu'il comporte les opérations successives de:
- obtention d'une réplique du moignon de la dent à reconstruire en revêtement réfractaire,
- application sur cette réplique d'une pièce de remodelage de la dent comprenant un mélange d'une poudre réfractaire et d'un liquide de dispersion,
- infiltration de la pièce de remodelage au moyen d'un métal en fusion, et
- élimination du revêtement réfractaire.

De façon avantageuse, la poudre réfractaire peut comporter, au choix, des grains:
- d'au moins un métal choisi parmi l'iridium, le ruthénium, le rhodium, le rhénium, l'osmium, le cobalt, le tungstène, le molybdène, le niobium, le tantale, le titane, le platine et le palladium et, éventuellement en plus, d'un métal choisi parmi l'or, l'argent, le zinc, l'indium et l'étain;
- d'un alliage à base d'un métal choisi parmi l'iridium, le ruthénium, le platine, le palladium, le rhodium, le cobalt, le tungstène, le molybdène, le niobium, le tantale et le titane;
- d'au moins un oxyde d'un métal choisi parmi l'aluminium, le zirconium, le titane, le magnésium et le silicium;
- d'au moins un nitrure d'un métal choisi parmi le titane, le tantale, le zirconium et le silicium;
- d'au moins un carbure d'un métal choisi parmi le titane, le tantale, le tungstène, le molybdène, le zirconium et le silicium;
- d'au moins un carbonitrure d'un métal choisi parmi le titane, le tantale, le zirconium et le silicium.

Les grains de poudre réfractaire ont une dimension moyenne inférieure à 100 µm mais, de préférence, inférieure à 50 µm.

Selon un premier mode de réalisation, l'étape d'infiltration comporte les opérations successives de:
- fixation sur la pièce de remodelage d'un paillon métallique possédant un point de fusion inférieur à celui de la poudre réfractaire,
- enrobage de la pièce dans un revêtement réfractaire, et
- cuisson de l'ensemble à une température supérieure au point de fusion du paillon mais inférieure au point de fusion de la poudre réfractaire pendant un temps suffisamment long pour permettre l'infiltration de la poudre par le métal fondu du paillon.

Selon un deuxième mode de réalisation, l'étape d'infiltration comporte les opérations successives de:
- première cuisson de l'ensemble à une température inférieure au point de fusion de la poudre réfractaire,
- application sur la pièce de remodelage d'une pâte métallique possédant un point de fusion inférieur à celui de la poudre réfractaire, et
- deuxième cuisson de l'ensemble à une température supérieure au point de fusion de ladite pâte métallique mais inférieure au point de fusion de la poudre réfractaire pendant un temps suffisamment long pour permettre l'infiltration de la poudre par le métal fondu de la pâte.

Selon un troisième mode de réalisation, l'étape d'infiltration comporte les opérations successives de:
- première cuisson de l'ensemble à une température inférieure au point de fusion de la poudre réfractaire,
- fixation sur la pièce de remodelage d'un paillon métallique possédant un point de fusion inférieur à celui de la poudre réfractaire, et
- deuxième cuisson de l'ensemble à une température supérieure au point de fusion du paillon mais inférieure au point de fusion de la poudre réfractaire pendant un temps suffisamment long pour permettre l'infiltration de la poudre par le métal fondu du paillon.

Selon un quatrième mode de réalisation, l'étape d'infiltration comporte les opérations successives de:
- prolongation de la pièce de remodelage par une languette de pompage,
- mise au contact de ladite languette d'un paillon métallique possédant un point de fusion inférieur à celui de la poudre réfractaire, et
- cuisson de l'ensemble à une température supérieure au point de fusion du paillon mais inférieure au point de fusion de la poudre réfractaire pendant un temps suffisamment long pour permettre l'infiltration de la poudre par le métal fondu du paillon.

Selon un cinquième mode de réalisation, l'étape d'infiltration comporte les opérations successives de:
- prolongation de la pièce de remodelage par une languette de pompage,
- première cuisson de l'ensemble à une température inférieure au point de fusion de la poudre réfractaire,
- mise au contact de ladite languette d'un paillon métallique possédant un point de fusion inférieur à celui de la poudre réfractaire, et
- deuxième cuisson de l'ensemble à une température supérieure au point de fusion du paillon mais inférieure au point de fusion de la poudre réfractaire pendant un temps suffisamment long pour permettre l'infiltration de la poudre par le métal fondu du paillon.

Avantageusement, le paillon ou la pâte métallique contient au moins un métal choisi parmi l'or, le cobalt, le fer, l'aluminium et le magnésium et, éventuellement en plus, au moins un métal choisi parmi l'argent, le chrome, le zinc, le cuivre, le gallium, le titane, le zirconium, l'étain, l'indium, le silicium et le tantale.

La présente invention concerne également une pièce de reconstruction dentaire obtenue par le procédé décrit ci-dessus. Elle est caractérisée en ce qu'elle comporte de 15 à 70 % de poudre réfractaire et respectivement de 85 à 30 % de métal infiltré dans le squelette poreux formé par ladite poudre.

L'invention sera mieux comprise à la lecture de la description qui suit, faite en regard du dessin annexé sur lequel:
- la figure 1 illustre les premières opérations du procédé; et
- les figures 2 à 6 montrent la suite des opérations pour cinq modes différents de mise en oeuvre de l'invention.

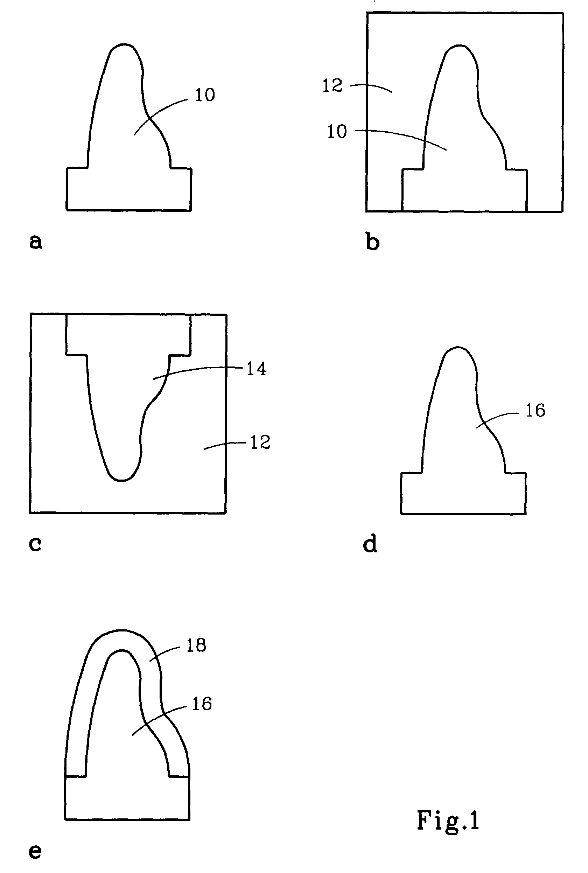

On se référera, tout d'abord, à la figure 1, qui illustre en 1a la première opération effectuée par le technicien pour fabriquer une reconstruction dentaire. Cette opération consiste à réaliser une réplique 10, en plâtre ou en résine, de l'empreinte effectuée par le dentiste dans la bouche du patient.

Puis, comme représenté en 1b, la réplique 10 du moignon de dent à reconstruire est enrobée dans un bloc de silicone 12 avant d'en être extraite pour former un moule dans lequel est versé un revêtement réfractaire liquide 14, comme montré en 1c. Ce revêtement peut être de tout type connu de l'homme de l'art, comme, par exemple, ceux qui sont commercialisés sous les références Multivest MS 24, Heravest Speed et Univest Rapid.

Après prise du revêtement et élimination du moule de silicone 12, le technicien dispose d'une réplique 16 du moignon de la dent en revêtement réfractaire, ainsi que représenté en 1d.

L'étape suivante du procédé, illustrée en 1e, est l'application sur la réplique en revêtement réfractaire 16 et son modelage, selon toute forme désirée, d'une pâte 18 réalisée par le mélange d'une poudre réfractaire et d'un liquide de dispersion. Il s'agit d'une poudre métallique ou céramique dont les grains ont une dimension moyenne inférieure à 100 µm mais, de préférence, inférieure à 50 µm.

Lorsque la poudre est métallique, elle est formée de grains d'un élément choisi parmi l'iridium, le ruthénium, le rhodium, le rhénium, l'osmium, le cobalt, le tungstène, le molybdène, le niobium, le tantale, le titane, le platine et le palladium. Elle peut aussi être constituée d'un mélange de grains des éléments précités entre eux ou avec d'autres éléments, tels que l'or, l'argent, le zinc, l'indium et l'étain. La poudre peut également être une poudre d'un alliage à base des mêmes métaux.

Lorsque la poudre est céramique, elle est formée de grains de soit un oxyde d'un métal choisi parmi l'aluminium, le zirconium, le titane, le magnésium et le silicium, soit un nitrure d'un métal choisi parmi le titane, le tantale, le zirconium et le silicium, soit un carbure d'un métal choisi parmi le titane, le tantale, le tungstène, le molybdène, le zirconium et le silicium, soit un carbonitrure d'un métal choisi parmi le titane, le tantale, le zirconium et le silicium, soit enfin de plusieurs de ces composants.

Le liquide de dispersion est avantageusement constitué d'un solvant, tel que l'eau ou le terpinéol, et d'un liant organique pouvant être éliminé par chauffage à une température supérieure à 600 °C, tel que le méthyle cellulose ou l'éthyle cellulose. Le liquide peut aussi contenir uniquement un liant organique, tel que de la cire ou le polyéthylène glycol.

Typiquement, à titre d'exemple non limitatif, la pâte de remodelage 18 est un mélange comportant :
- 30 à 40 % en volume soit d'un mélange comprenant 70 % en poids de poudre d'iridium et 30 % en poids de poudre d'or, soit de 100 % en poids de poudre de molybdène ou de tungstène; et
- 70 à 60 % en volume d'une solution contenant 99 % en poids d'eau et 1% en poids de méthyle cellulose.

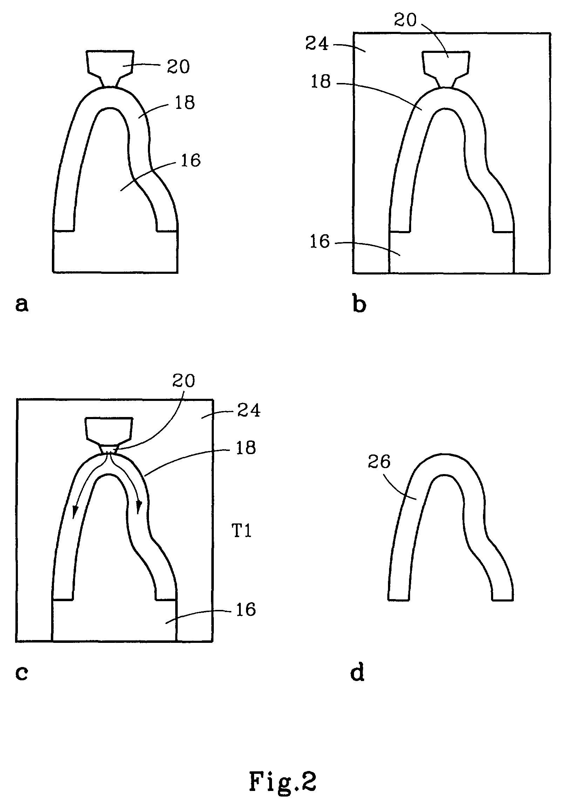

La figure 2 illustre la suite des opérations du procédé selon un premier mode avantageux de mise en oeuvre de l'invention.

L'opération montrée en 2a est la mise en place, au sommet de la pièce de remodelage 18, d'un paillon métallique 20 possédant un point de fusion inférieur à celui de la pièce 18 de, typiquement plus de 100 °C. Le paillon 20 est collé à la pièce à l'aide d'une pâte similaire à la pâte de remodelage 18 précédemment décrite.

Le paillon 20 est avantageusement constitué d'un métal choisi parmi l'or, le cobalt, le fer, l'aluminium et le magnésium ou d'un alliage à base de ces métaux. Il peut aussi contenir des additifs choisis parmi l'argent, le chrome, le zinc, le cuivre, le gallium, le titane, le zirconium, l'étain, l'indium, le silicium et le tantale.

Typiquement, lorsque la pâte de remodelage a la composition donnée ci-dessus à titre d'exemple, le paillon 20 est un alliage contenant 90 % en poids d'or et 10 % en poids d'argent.

Après séchage, lorsque seule subsiste donc la poudre réfractaire qui forme ainsi un squelette poreux, l'étape suivante, illustrée par la figure 2b, est l'enrobage de l'ensemble dans un revêtement réfractaire liquide 24 semblable au revêtement 16 précédemment décrit.

Lorsque le revêtement 24 s'est solidifié, comme illustré par la figure 2c, le tout est cuit à une température T1 supérieure au point de fusion du paillon 20 mais inférieure au point de fusion de la pièce 18 pendant un temps suffisamment long pour permettre l'infiltration, par capillarité, de ce squelette poreux par le métal fondu du paillon.

Typiquement, lorsque la pâte de remodelage et le paillon ont les compositions données ci-dessus, la cuisson est faite à 1150° C pendant 20 minutes dans un four de laboratoire sous air, tel que celui commercialisé sous la référence Kavo EWL 5646 (Kavo, DE), ou un four pour la cuisson de céramique dentaire sous air ou sous vide, tel que celui commercialisé sous la référence Programat P95 (Ivoclar, LI).

Il ne reste plus, alors, qu'à laisser refroidir la pièce puis d'éliminer les revêtements réfractaires 16 et 24 ainsi que l'éventuel résidu du paillon 20. Ce qui subsiste est une pièce de reconstruction 26, représentée à la figure 2d, de même forme que la pièce 18, qui peut être travaillée, de manière conventionnelle, en vue de sa mise en bouche.

La poudre réfractaire formant le squelette de cette pièce de reconstruction 26 a une porosité qui peut, selon la morphologie et la distribution de taille des grains des poudres, peut être comprise entre environ 40 et 70 %, c'est à dire que 40 à 70 % du volume est occupé par le métal infiltré et donc que respectivement les 60 à 30 % restants sont occupés par la poudre. On peut cependant modifier cette proportion en mélangeant à la poudre réfractaire une poudre métallique ayant une composition similaire à celle du paillon.

Dans ce cas, lors de la cuisson, cette poudre métallique fond et s'allie au métal infiltré, de sorte qu'il est alors possible d'obtenir une reconstruction contenant, typiquement, de 15 à 70 % du volume en poudre réfractaire et, respectivement, de 85 à 30 % du volume en métal ayant la composition du métal d'infiltration.

Il est ainsi possible de réaliser une reconstruction dentaire dont la composition et les teneurs respectives en éléments non précieux, semi-précieux et précieux, peuvent être choisies quasiment à la carte dans une large palette.

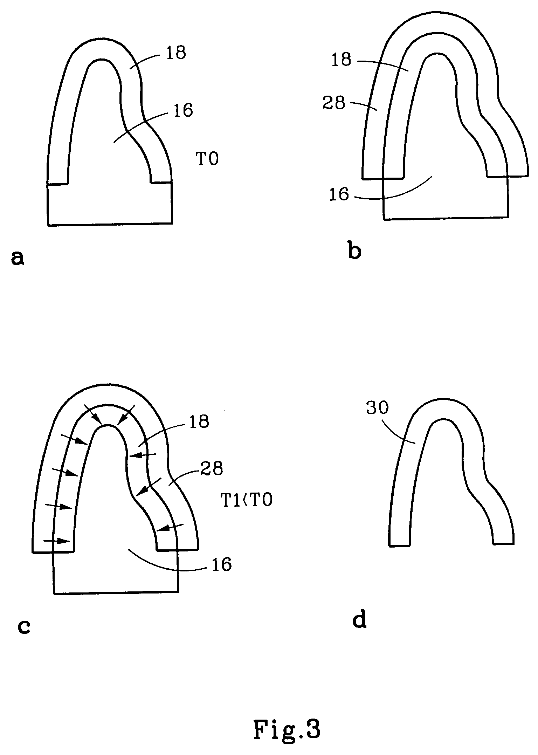

On se référera maintenant à la figure 3 qui, selon un deuxième mode avantageux de mise en oeuvre de l'invention, montre les opérations effectuées après l'application sur la réplique 16 d'une pâte de remodelage 18 à base de poudre réfractaire, comme illustré par la figure 1e.

Dans ce cas, la pièce est, tout d'abord, comme montré sur la figure 3a, cuite à une température To inférieure au point de fusion de la pâte 18 pendant un temps suffisamment long pour transformer celle-ci en un squelette poreux.

Comme illustré par le figure 3b, la pièce 18 est alors recouverte d'une deuxième couche de pâte métallique 28 ayant une composition identique à celle du paillon 20 utilisé dans le premier mode de réalisation.

La pièce subit ensuite, ainsi qu'illustré par la figure 3c, une deuxième cuisson à une température T1 supérieure au point de fusion de la pâte 28 mais inférieure au point de fusion du squelette poreux 18 pendant un temps suffisamment long pour permettre son infiltration par capillarité. Cette opération est typiquement effectuée dans des conditions identiques à celles de l'opération illustrée par la figure 2b. De préférence, la température T1 de cette deuxième cuisson est inférieure de plus de 100 °C à celle To de la première.

Il ne reste plus, alors, qu'à laisser refroidir la pièce puis à éliminer le revêtement réfractaire 16 en le rompant au marteau. Ce qui subsiste est une pièce de reconstruction 30, représentée à la figure 3d, de même forme que la pièce 18, qui peut être travaillée, de manière conventionnelle, en vue de sa mise en bouche.

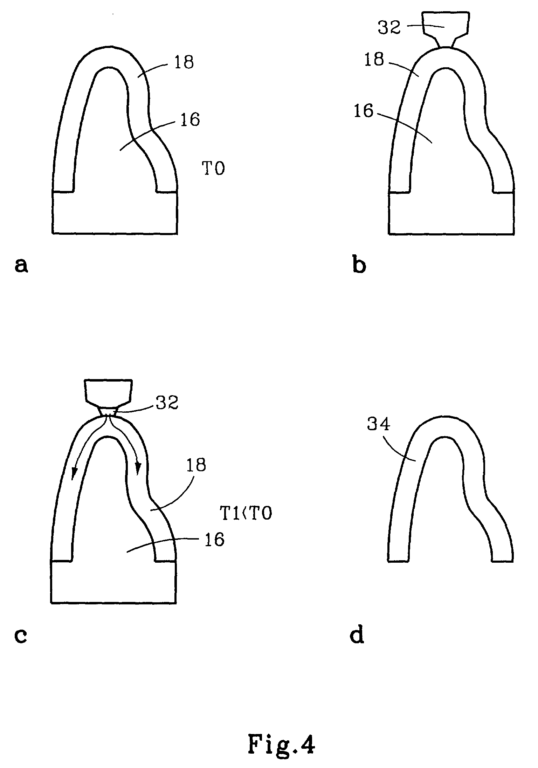

La figure 4 illustre un troisième mode de réalisation du procédé selon l'invention, assez semblable à celui de la figure 3. Dans ce cas, après l'opération de première cuisson de la figure 4a, identique à celle de la figure 3a, on met en place au sommet de la pièce de remodelage 18, comme illustré par la figure 4b, un paillon métallique 32 identique au paillon 20 de la figure 2a.

La pièce subit ensuite, ainsi qu'illustré par la figure 4c, une deuxième cuisson à une température T1 supérieure au point de fusion du paillon 32 mais inférieure au point de fusion du squelette poreux 18 pendant un temps suffisamment long pour permettre son infiltration par capillarité. Cette opération est effectuée dans des conditions identiques à celles de l'opération illustrée par la figure 3c.

Après refroidissement de la pièce, le revêtement réfractaire 16 est éliminé ainsi qu'un éventuel résidu du paillon 32, pour laisser subsister une pièce de reconstruction 34, représentée à la figure 4d, de même forme que la pièce 18.

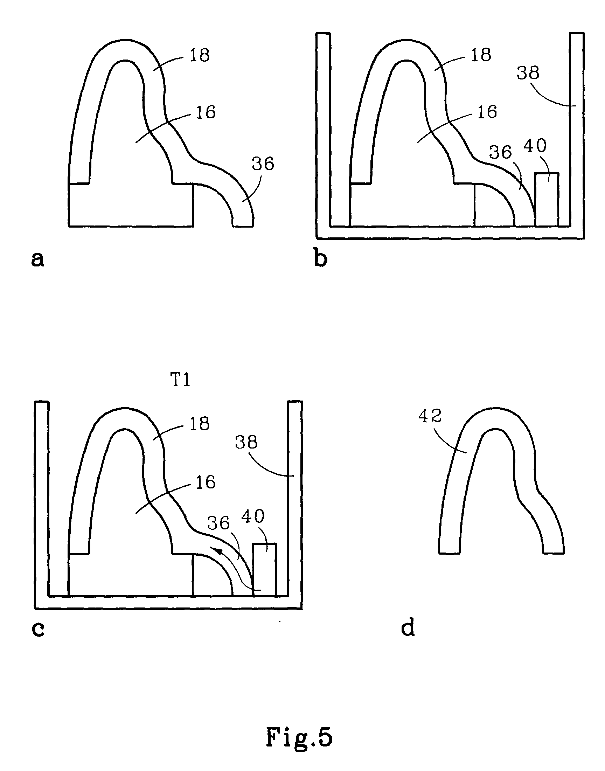

Selon un quatrième mode avantageux de mise en oeuvre de l'invention, la figure 5 montre les opérations effectuées après l'application sur la réplique 16 d'une pâte de remodelage 18 à base de poudre réfractaire, comme illustré par la figure 1e.

La première opération, représentée par la figure 5a, est le prolongement de la pâte 18 par une languette de pompage 36 de même composition qu'elle.

La pièce est ensuite déposée dans un bac de fusion 38, montré à la figure 5b, en mettant la languette 36 au contact d'un paillon métallique 40 de même composition que le paillon 20 de la figure 2a.

L'opération suivante, illustrée par la figure 5c, est la cuisson de l'ensemble à une température T1 supérieure au point de fusion du paillon 40 mais inférieure au point de fusion du squelette poreux 18 pendant un temps suffisamment long pour permettre son infiltration par capillarité au travers de la languette de pompage 36.

Après refroidissement de la pièce, la dernière opération consiste à éliminer le revêtement réfractaire 16 ainsi que la languette 36 et un éventuel résidu du paillon 40 pour laisser subsister une pièce de reconstruction 42, représentée à la figure 5d, de même forme que la pièce 18.

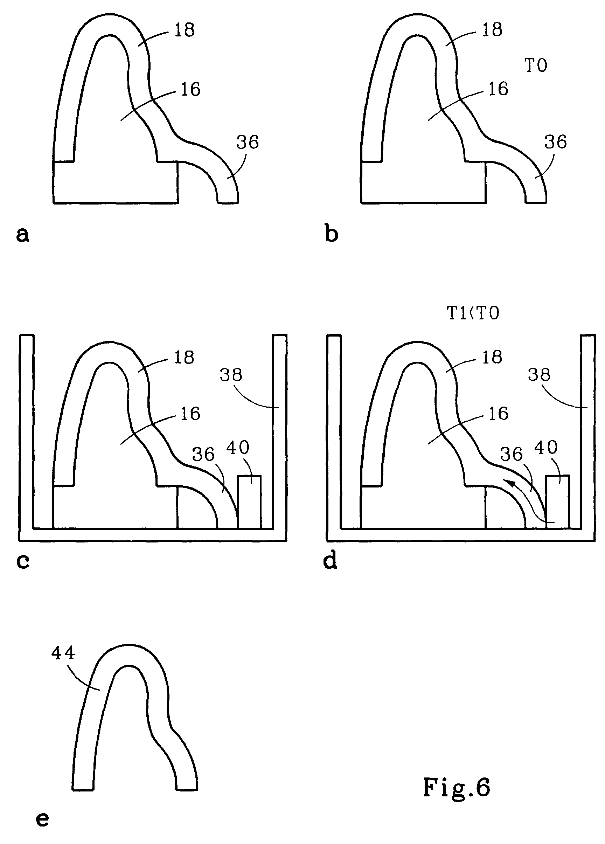

On se référera, pour terminer, à la figure 6 qui représente, selon un cinquième mode avantageux de mise en oeuvre de l'invention, les opérations effectuées après l'application sur la réplique 16 d'une pâte de remodelage 18 à base de poudre réfractaire, comme illustré par la figure 1e.

La première opération, montrée sur la figure 6a, est identique à celle de la figure 5a.

L'opération suivante, représentée sur la figure 6b et identique aux opérations des figures 3a et 4a, consiste à cuire la pièce à une température To inférieure au point de fusion de la pâte 18 pendant un temps suffisamment long pour transformer celle-ci en un squelette poreux.

Les deux opérations suivantes, illustrées par les figures 6c et 6d, sont identiques aux opérations des figures 5b et 5c, la température T1 de la deuxième cuisson étant, comme dans le cas de la figure 3c, inférieure de plus de 100 °C à celle To de la première.

Enfin, après refroidissement de la pièce, la dernière opération consiste à éliminer le revêtement réfractaire 16 ainsi que la languette 36 et un éventuel résidu du paillon 40 pour laisser subsister une pièce de reconstruction 44, représentée à la figure 6d, de même forme que la pièce 18.

## Revendications

1. Procédé de réalisation d'une reconstruction dentaire, **caractérisé en ce qu'**il comporte les étapes successives de:
- obtention d'une réplique (16) du moignon de dent à reconstruire en revêtement réfractaire,
- application sur ladite réplique d'une pièce de remodelage (18) de la dent comprenant un mélange d'une poudre réfractaire et d'un liquide de dispersion,
- infiltration de ladite pièce (18) au moyen d'un métal en fusion, et
- élimination du revêtement réfractaire (16).

2. Procédé selon la revendication 1, **caractérisé en ce que** ladite poudre réfractaire comporte des grains d'au moins un métal choisi parmi l'iridium, le ruthénium, le rhodium, le rhénium, l'osmium, le cobalt, le tungstène, le molybdène, le niobium, le tantale, le titane, le platine et le palladium.

3. Procédé selon la revendication 2, **caractérisé en ce que** ladite poudre réfractaire comporte, en plus, des grains d'un métal choisi parmi l'or, l'argent, le zinc, l'indium et l'étain.

4. Procédé selon la revendication 1, **caractérisé en ce que** ladite poudre comporte des grains d'un alliage à base d'un métal choisi parmi l'iridium, le ruthénium, le platine, le palladium, le rhodium, le cobalt, le tungstène, le molybdène, le niobium, le tantale et le titane.

5. Procédé selon la revendication 1, **caractérisé en ce que** ladite poudre réfractaire comporte des grains d'au moins un oxyde d'un métal choisi parmi l'aluminium, le zirconium, le titane, le magnésium et le silicium.

6. Procédé selon la revendication 1, **caractérisé en ce que** ladite poudre réfractaire comporte des grains d'au moins un nitrure d'un métal choisi parmi le titane, le tantale, le zirconium et le silicium.

7. Procédé selon la revendication 1, **caractérisé en ce que** ladite poudre réfractaire comporte des grains d'au moins un carbure d'un métal choisi parmi le titane, le tantale, le tungstène, le molybdène, le zirconium et le silicium.

8. Procédé selon la revendication 1, **caractérisé en ce que** ladite poudre réfractaire comporte des grains d'au moins un carbonitrure d'un métal choisi parmi le titane, le tantale, le zirconium et le silicium.

9. Procédé selon la revendication 1, **caractérisé en ce que** les grains de ladite poudre réfractaire ont une dimension moyenne inférieure à 100 µm.

10. Procédé selon la revendication 1, **caractérisé en ce que** l'étape d'infiltration comporte les opérations successives de:
- fixation sur la pièce de remodelage (18) d'un paillon métallique (20) possédant un point de fusion inférieur à celui de la poudre réfractaire,
- enrobage de la pièce dans un revêtement réfractaire (24), et
- cuisson de l'ensemble à une température (T1) supérieure au point de fusion du paillon mais inférieure au point de fusion de la poudre réfractaire pendant un temps suffisamment long pour permettre l'infiltration de la poudre par le métal fondu du paillon (20).

11. Procédé selon la revendication 1, **caractérisé en ce que** l'étape d'infiltration comporte les opérations successives de:
- première cuisson de l'ensemble à une température (To) inférieure au point de fusion de la poudre réfractaire,
- application sur la pièce de remodelage (18) d'une pâte métallique (28) possédant un point de fusion inférieur à celui de la poudre réfractaire, et
- deuxième cuisson de l'ensemble à une température (T1) supérieure au point de fusion de ladite pâte métallique mais inférieure au point de fusion de la poudre réfractaire pendant un temps suffisamment long pour permettre l'infiltration de la poudre par le métal fondu de la pâte (28).

12. Procédé selon la revendication 1, **caractérisé en ce que** l'étape d'infiltration comporte les opérations successives de:
- première cuisson de l'ensemble à une température (To) inférieure au point de fusion de la poudre réfractaire,
- fixation sur la pièce de remodelage (18) d'un paillon métallique (32) possédant un point de fusion inférieur à celui de la poudre réfractaire, et
- deuxième cuisson de l'ensemble à une température (T1) supérieure au point de fusion du paillon mais inférieure au point de fusion de la poudre réfractaire pendant un temps suffisamment long pour permettre l'infiltration de la poudre par le métal fondu du paillon (32).

13. Procédé selon la revendication 1, **caractérisé en ce que** l'étape d'infiltration comporte les opérations successives de:
- prolongation de la pièce de remodelage (18) par une languette de pompage (36),
- mise au contact de ladite languette d'un paillon métallique (40) possédant un point de fusion inférieur à celui de la poudre réfractaire, et
- cuisson de l'ensemble à une température (T1) supérieure au point de fusion du paillon mais inférieure au point de fusion de la poudre réfractaire pendant un temps suffisamment long pour permettre l'infiltration de la poudre par le métal fondu du paillon (40).

14. Procédé selon la revendication 1, **caractérisé en ce que** l'étape d'infiltration comporte les opérations successives de:
- prolongation de la pièce de remodelage (18) par une languette de pompage (36),
- première cuisson de l'ensemble à une température (To) inférieure au point de fusion de la poudre réfractaire,
- mise au contact de ladite languette d'un paillon métallique (40) possédant un point de fusion inférieur à celui de la poudre réfractaire, et
- deuxième cuisson de l'ensemble à une température (T1) supérieure au point de fusion du paillon mais inférieure au point de fusion de la poudre réfractaire pendant un temps suffisamment long pour permettre l'infiltration de la poudre par le métal fondu du paillon (40).

15. Procédé selon l'une des revendications 10 à 14, **caractérisé en ce que** le paillon (20, 32, 40) ou la pâte métallique (28) contient au moins un métal choisi parmi l'or, le cobalt, le fer, l'aluminium et le magnésium.

16. Procédé selon la revendication 15, **caractérisé en ce que** le paillon (20, 32, 40) ou la pâte métallique (28) contient, en plus, au moins un métal choisi parmi l'argent, le chrome, le zinc, le cuivre, le gallium, le titane, le zirconium, l'étain, l'indium, le silicium et le tantale.

17. Pièce de reconstruction dentaire obtenue par le procédé selon l'une quelconque des revendications 1 à 16, **caractérisée en ce qu'**elle comporte de 15 à 70 % de poudre réfractaire et respectivement de 85 à 30 % de métal infiltré dans le squelette poreux formé par ladite poudre.
